(19) Europäisches Patentamt
European Patent Office
Office européen des brevets

(11) Veröffentlichungsnummer: **0 082 398**
**B1**

(12) # EUROPÄISCHE PATENTSCHRIFT

(45) Veröffentlichungstag der Patentschrift: **27.03.85**

(51) Int. Cl.⁴: **C 07 D 275/04**, C 07 C 145/00

(21) Anmeldenummer: **82111291.9**

(22) Anmeldetag: **06.12.82**

(54) **Verfahren zur Herstellung von 1,2-Benzisothiazolin-3-onen.**

(30) Priorität: **21.12.81 DE 3150629**

(43) Veröffentlichungstag der Anmeldung:
**29.06.83 Patentblatt 83/26**

(45) Bekanntmachung des Hinweises auf die Patenterteilung:
**27.03.85 Patentblatt 85/13**

(84) Benannte Vertragsstaaten:
**BE CH DE FR GB LI**

(56) Entgegenhaltungen:
**GB - A - 848 130**
**US - A - 3 300 378**

**THE CHEMISTRY OF HETEROCYCLIC COMPOUNDS, Band 4, 1952, Seiten 253-258, Interscience Publishers, Inc., New York, USA, L.L. BAMBAS: "Five-membered heterocyclic compounds with nitrogen and sulfur or nitrogen, sulfur, and oxygen (except thiazole)"**

(73) Patentinhaber: **BASF Aktiengesellschaft, Carl-Bosch-Strasse 38, D-6700 Ludwigshafen (DE)**

(72) Erfinder: **Hagen, Helmut, Dr., Max-Slevogt-Strasse 17e, D-6710 Frankenthal (DE)**
Erfinder: **Ziegler, Hans, Dr., Am Speyerweg 48, D-6704 Mutterstadt (DE)**

**EP 0 082 398 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 1,2-Benzisothiazolin-3-onen durch Umsetzung von Benz-1,2-dithiol-3-thionen mit Chlor und Wasser und dann mit Ammoniak oder Aminen in Gegenwart inerter Lösungsmittel.

Es ist bekannt, 1,2-Benzisothiazolin-3-one durch Chlorierung von Dithiodibenzoylsäurechloriden und folgende Umsetzung mit Aminen herzustellen (US-PS 2 870 015).

Eine weitere Methode (Reissert und Manns, Ber. 61, Seiten 1308 bis 1316 [1928]) ist die Umsetzung von Natronlauge mit Dithiosalicylsäureamiden (T. Vitali, L. Amoretti und V. Plarzi, Farmaco. Ed. Sci. 23, 1075 [1968]) oder aus Benzoesäure-2-sulfenamiden durch Cyclisierung bei 0 bis 210°C (Offenlegungsschrift 2 119 730). Man kann auch 2-Halogensulfidbenzoylhalogenide mit Diaminen umsetzen (belgische Patentschrift 617 384).

Diese Verfahren zur Darstellung von 1,2-Benzisothiazolin-3-onen befriedigen jedoch wegen der ungünstigen Herstellung der Dithiosalicylsäuren, die über mehrere Stufen verläuft, und somit bezüglich der Wirtschaftlichkeit des Verfahrens nicht.

Es wurde nun gefunden, daß man 1,2-Benzisothiazolin-3-one der Formel

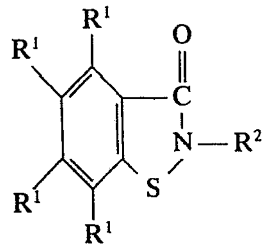

(I)

worin die einzelnen Reste $R^1$ gleich oder verschieden sein können und jeweils ein Wasserstoffatom, einen aliphatischen Rest, eine aromatische Acylgruppe, eine Nitrogruppe oder ein Halogenatom bedeuten, 2 benachbarte Reste $R^1$ auch zusammen mit den 2 benachbarten Kohlenstoffatomen Glieder eines anellierten aromatischen Ringes bezeichnen können, und $R^2$ für ein Wasserstoffatom, einen aliphatischen, cycloaliphatischen, araliphatischen oder aromatischen Rest steht, vorteilhaft erhält, wenn man Benz-1,2-dithiol-3-thione der Formel

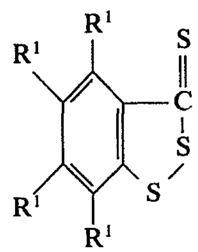

(II)

worin $R^1$ die vorgenannte Bedeutung besitzt,

a) mit Chlor und Wasser in Gegenwart von unter den Reaktionsbedingungen inerten organischen Lösungsmitteln umsetzt und
b) die so erhaltenen 2-Chlorthio-arylcarbonsäurechloride der Formel

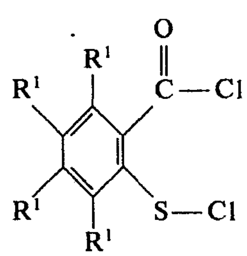

(III)

worin $R^1$ die vorgenannte Bedeutung besitzt, mit Ammoniak oder einem primären Amin der Formel

$$H_2N—R^2 \qquad (IV)$$

worin $R^2$ die vorgenannte Bedeutung besitzt, in Gegenwart eines unter den Reaktionsbedingungen inerten, organischen Lösungsmittels umsetzt.

Die Umsetzung kann für den Fall der Verwendung von Benz-1,2-dithiol-3-thion und Ammoniak durch die folgenden Formeln wiedergegeben werden:

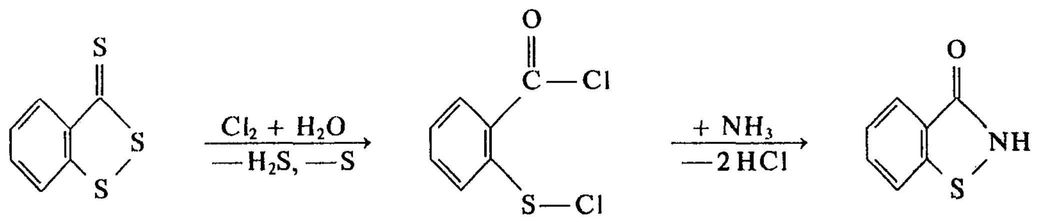


Im Vergleich zu den bekannten Verfahren geht das Verfahren nach der Erfindung von leichter zugänglichen Ausgangsstoffen aus und liefert überraschend auf einfachere und wirtschaftlichere Weise 1,2-Benzisothiazolin-3-one in bessere Raum-Zeit-Ausbeute, Ausbeute und Reinheit. Die Ausgangsstoffe Benz-1,2-dithiol-3-thione sind einfacher und wirtschaftlicher und auch mit einer größeren Anzahl möglicher Substituenten zugänglich, da sie in hoher Ausbeute und Reinheit aus 2-Halogenbenzylhalogeniden mit Schwefel bei 50—200°C in Gegenwart von Monoalkylglykoläthern und Alkalialkanolat hergestellt werden können. Alle diese vorteilhaften Ergebnisse sind im Hinblick auf den Stand der Technik überraschend.

Bevorzugte Ausgangsstoffe II, IV und Stoffe III und dementsprechend bevorzugte 1,2-Benzisothiazol-3-one I sind solche, in deren Formeln die einzelnen Reste $R^1$ gleich oder verschieden sein können und jeweils ein Wasserstoffatom, einen Alkylrest mit 1 bis 6 Kohlenstoffatomen, ein Bromatom, ein Chloratom, eine Arylcarbonylgruppe mit 7 bis 18 Kohlenstoffatomen, insbesondere eine Benzoyl- oder Naphthoylgruppe oder eine Nitrogruppe bedeuten, 2 benachbarte Reste $R^1$ auch zusammen mit den 2 benachbarten Kohlenstoffatomen Glieder eines anellierten Benzols oder Naphthalins bezeichnen können, $R^2$ für ein Wasserstoffatom, einen Alkylrest mit 1 bis 6 Kohlenstoffatomen, einen Alkenyl- oder Alkinylrest mit jeweils 2 bis 6 Kohlenstoffatomen, wobei die Alkyl-, Alkenyl- und Alkinylgruppen noch durch Hydroxy-, Alkoxygruppen, Dialkylaminogruppen mit jeweils 1—4 Kohlenstoffatomen je Alkylgruppe, Halogenatome, insbesondere Chloratome, substituiert sein können, einen Cycloalkylrest mit 5—8 Kohlenstoffatomen, einen Aralkylrest oder Alkylarylrest mit jeweils 7—12 Kohlenstoffatomen, einen Phenylrest steht, wobei die Aralkylreste, Alkylarylreste und der Phenylrest an der Arylgruppe noch durch Halogene, insbesondere Chlor oder Brom, Nitro-, Trichlormethyl-, Trifluormethylgruppen, Alkylgruppen, Dialkylaminogruppen mit jeweils 1—4 Kohlenstoffatomen je Alkylgruppe substituiert sein können. Die vorgenannten Reste können noch durch unter den Reaktionsbedingungen inerte Gruppen und/oder Atome, z. B. Alkylgruppen oder Alkoxygruppen mit jeweils 1 bis 4 Kohlenstoffatomen, substituiert sein.

Die Umsetzung wird im allgemeinen in beiden Stufen a) und b) bei einer Temperatur von 0 bis 80°C, vorzugsweise bei 15 bis 35°C, drucklos oder unter Druck, kontinuierlich oder diskontinuierlich durchgeführt. Man verwendet unter den Reaktionsbedingungen inerte Lösungsmittel. Als Lösungsmittel kommen z. B. in Frage: aromatische Kohlenwasserstoffe, z. B. Toluol, Ethylbenzol, o-, m-, p-Xylol, Isopropylbenzol, Methylnaphthalin; Halogenkohlenwasserstoffe, insbesondere Chlorkohlenwasserstoffe, z. B.

> Tetrachlorethylen, 1,1,2,2- oder 1,1,1,2-Tetrachlorethan, Dichlorpropan, Methylenchlorid, Dichlorbutan, Isopropylbromid, n-Propylbromid, Butylbromid, Chloroform, Ethyljodid, Propyljodid, Tetrachlorkohlenstoff, 1,1,1- oder 1,1,2-Trichlorethan, Trichlorethylen, Pentachlorethan, 1,2-Dichlorethan, 1,1-Dichlorethan, n-Propylchlorid, 1,2-cis-Dichlorethylen, n-Butylchlorid, 2-, 3-und iso-Butylchlorid;

und entsprechende Gemische. Zweckmäßig verwendet man das Lösungsmittel in beiden Stufen jeweils in einer Menge von 200 bis 10 000 Gewichtsprozent, vorzugsweise von 50 bis 1000 Gewichtsprozent, bezogen auf Ausgangsstoff II.

Chlor und Wasser werden mit Ausgangsstoff II in stöchiometrischer Menge oder im Überschuß, zweckmäßig in einem Verhältnis von 3 bis 10, vorzugsweise 3,5 bis 5 Mol Chlor und/oder von 1,5 bis 1000, vorzugsweise 1,5 bis 10, insbesondere 1,9 bis 2,2 Mol Wasser je Ausgangsstoff II umgesetzt. Das Chlor wird vorzugsweise gasförmig in die Reaktionslösung eingeleitet. Das primäre Amin IV oder Ammoniak wird mit dem Ausgangsstoff III in stöchiometrischer Menge oder im Überschuß, zweckmäßig in einem Verhältnis von 1 bis 10, insbesondere 1 bis 3 Mol Amin IV oder $NH_3$ je Mol Ausgangsstoff II in Stufe b) umgesetzt. Die Umsetzung wird zweckmäßig mit 18- bis 31gewichtsprozentiger, vorzugsweise 20- bis 26gewichtsprozentiger, wäßriger Ammoniaklösung durchgeführt. Es kann auch bei Stufe b) in Gegenwart einer Protonen bindenden Verbindung gearbeitet werden. Hierzu kommen z. B. tert.-Amine, wie Triethylamin oder Pyridin in Frage. Diese Basen werden in stöchiometrischer Menge oder im Überschuß, zweckmäßig in einem Verhältnis von 2 bis 10, insbesondere 2 bis 3 Mol Base je Mol Ausgangsstoff II umgesetzt.

Die Reaktion kann wie folgt durchgeführt werden: Ausgangsstoff II, Chlor, Wasser und Lösungsmittel werden in einem Reaktor während 10 bis 50 Stunden bei den vorgenannten Reaktionstemperaturen miteinander umgesetzt. Anschließend werden, zweckmäßig nach Entfernung des Lösungsmittels, die Ausgangsstoffe III und IV in Gegenwart von inertem Lösungsmittel während 6 bis 25 Stunden bei der Reaktionstemperatur umgesetzt.

Der Endstoff wird aus dem Reaktionsgemisch der Stufe b) in bekannter Weise, z. B. durch Absaugen und Wäsche des Endstoffs bzw. durch Absaugen, Extraktion des Festgutes mit einem säurebindenden Mittel, z. B. wäßrige Natronlauge, und Ansäuern des Extrakts, z. B. mit verdünnter Salzsäure, isoliert.

Die durch das Verfahren nach der Erfindung erhältlichen 1,2-Benzisothiazolin-3-one sind wertvolle Ausgangsstoffe für die Herstellung von Schädlingsbekämpfungsmitteln, Farbstoffen, Pharmazeutika. Insbesondere besitzen sie antibakterielle und fungizide Wirksamkeit. Bezüglich dieser Verwendung wird auf US-PS 3 065 123, DE-OS 2 419 017, GB-PS 976 028, US-PS 3 517 022, US-PS 3 761 489, US-PS 3 821 389, US-PS 3 862 955 verwiesen.

Es gibt ebenfalls Anwendungsmöglichkeiten im pharmazeutischen Bereich. So offenbaren z. B. die deutsche Offenlegungsschrift 2 652 201 die Anwendung der 1,2-Benzisothiazolin-3-one als Thrombosehemmer und die deutsche Offenlegungsschrift 2 629 018 die Anwendung als Spermizid. Auch in der Fotographie (US-PS 2 870 015) sind Verwendungsmöglichkeiten gegeben. BE-PS 565 380 beschreibt entsprechende antiphlogistische Verwendung. Im weiteren wird ebenfalls auf die vorgenannten Druckschriften im Stand der Technik verwiesen.

Beispiel 1

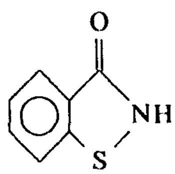

In eine Suspension von 736 Gramm Benz-1,2-dithiol-3-thion in 5000 Gramm Dichlormethan wurden bei 20—30°C 570 Gramm Chlorgas eingeleitet. Nach 2 Stunden bei 20—30°C wurden 144 Gramm Wasser zugegeben. Das Reaktionsgemisch wurde nun 6 Stunden bei 20—30°C gerührt. Anschließend leitete man 570 Gramm Chlor bei 10 bis 20°C ein, beließ das Gemisch 24 Stunden bei 25°C und entfernte die leichtflüchtigen Bestandteile im Vakuum.

Das so gewonnene 2-Chlorthio-benzoesäurechlorid ($Kp_{1\,bar}$: 130°C) wurde in 3000 Gramm Toluol gelöst und bei 20—25°C mit 1000 Gramm einer 25gewichtsprozentigen wäßrigen Ammoniaklösung versetzt. Nach 12 Stunden wurde das Gemisch abgesaugt und der Niederschlag mit Wasser gewaschen.

Man erhält 512 g 1,2-Benzisothiazolin-3-on (entsprechend 85% der Theorie) vom Schmelzpunkt 157—158°C.

Beispiel 2

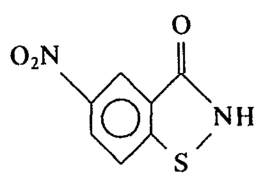

In eine Suspension von 916 Gramm 5-Nitrobenz-1,2-dithiol-3-thion in 6000 Gramm Dichlormethan wurden bei 20—30°C 570 Gramm Chlor eingeleitet. Nach 2 Stunden wurden 144 Gramm Wasser zugegeben. Das Reaktionsgemisch wurde 12 Stunden bei 20—30°C gerührt. Danach wurden 570 Gramm Chlor bei 10 bis 20°C eingeleitet und die Reaktionslösung bei 20—30°C 24 Stunden gerührt. Nach Filtration wurden die leichtflüchtigen Bestandteile im Vakuum entfernt.

Das so gewonnene 2-Chlorthio-5-nitrobenzoesäurechlorid wurde in 3000 Gramm Methylenchlorid gelöst und bei 20 bis 25°C mit 1000 Gramm 25gewichtsprozentiger Ammoniaklösung versetzt. Nach 24 Stunden wurde das Gemisch abgesaugt und der Niederschlag mit verdünnter wäßriger Natronlauge extrahiert. Aus der wäßrigen Lösung wurde der Endstoff I durch Ansäuern mit verdünnter Salzsäure wieder ausgefällt. Nach dem Trocknen erhielt man 710 Gramm (entsprechend 90% der Theorie) 5-Nitro-1,2-benzisothiazolin-3-on mit einem Schmelzpunkt von 280°C.

Beispiel 3

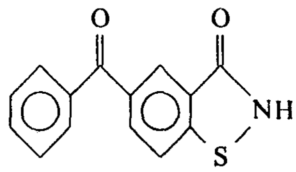

1152 Gramm 5-Benzoylbenz-1,2-dithiol-3-thion wurden analog Beispiel 1 umgesetzt. Man erhielt 691 Gramm 5-Benzoyl-1,2-benzisothiazolin-3-on (entsprechend 68% der Theorie) vom Schmelzpunkt 233°C.

Beispiel 4

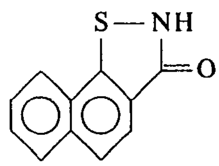

936 Gramm Naphtho-(1,2)-1,2-dithiol-3-thion analog Beispiel 1 umgesetzt. Man erhielt 520 Gramm Naphtho-(1,2)-1,2-benzisothiazolin-3-on (entsprechend 65% der Theorie) vom Schmelzpunkt 201°C.

Beispiel 5

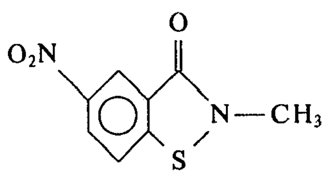

916 Gramm 5-Nitrobenz-1,2-dithiol-3-thion wurden analog Beispiel 2 zu 2-Chlorthio-5-nitrobenzoesäurechlorid umgesetzt, das in 5000 Gramm Diethylether gelöst wurde. In diese Lösung wurden bei 20 bis 25°C 400 Gramm Methylamin gasförmig eingeleitet. Nach 24 Stunden wurde das Gemisch abgesaugt und der Niederschlag mit Wasser gewaschen. Nach Trocknen erhielt man 663 Gramm entsprechend 79% der Theorie 2-Methyl-5-nitrobenzisothiazolin-3-on vom Schmelzpunkt 210°C.

Beispiel 6

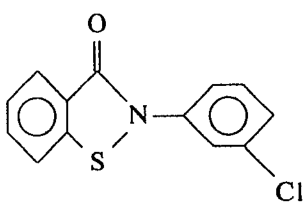

736 Gramm Benz-1,2-dithiol-3-thion wurden analog Beispiel 1 zu 2-Chlorthiobenzoesäurechlorid umgesetzt. Das so gewonnene 2-Chlorthiobenzoesäurechlorid wurde gelöst in 2000 Gramm Dichlormethan, bei 10 bis 20°C zu einer Lösung von 510 Gramm 3-Chloranilin und 808 Gramm Triethylamin in 6000 Gramm Dichlormethan zugetropft. Nach 12 Stunden wurde die Lösung mit Wasser extrahiert und eingeengt. Aus Methanol erhielt man 720 Gramm 2-(3'-Chlorphenyl)-benzisothiazolin-3-on entsprechend 69% der Theorie vom Schmelzpunkt 138°C.

**Patentanspruch**

Verfahren zur Herstellung von 1,2-Benzisothiazolin-3-onen der Formel

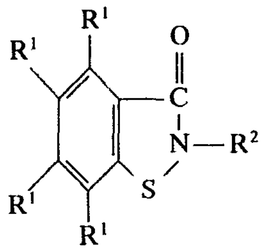

(I)

worin die einzelnen Reste $R^1$ gleich oder verschieden sein können und jeweils ein Wasserstoffatom, einen aliphatischen Rest, eine aromatische Acylgruppe, eine Nitrogruppe oder ein Halogenatom bedeuten, 2 benachbarte Reste $R^1$ auch zusammen mit den 2 benachbarten Kohlenstoffatomen Glieder eines anellierten aromatischen Ringes bezeichnen können, und $R^2$ für ein Wasserstoffatom, einen aliphatischen, cycloaliphatischen, araliphatischen oder aromatischen Rest steht, dadurch gekennzeichnet, daß man Benz-1,2-dithiol-3-thione der Formel

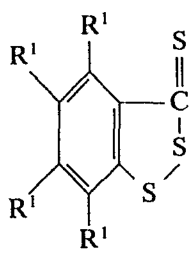

(II)

worin $R^1$ die vorgenannte Bedeutung besitzt,

a) mit Chlor und Wasser in Gegenwart von unter den Reaktionsbedingungen inerten organischen Lösungsmitteln umsetzt und
b) die so erhaltenen 2-Chlorthio-arylcarbonsäurechloride der Formel

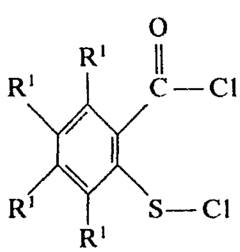

(III)

worin $R^1$ die vorgenannte Bedeutung besitzt, mit Ammoniak oder einem primären Amin der Formel

$$H_2N-R^2 \qquad (IV)$$

worin $R^2$ die vorgenannte Bedeutung besitzt, in Gegenwart eines unter den Reaktionsbedingungen inerten, organischen Lösungsmittels umsetzt.

**Claim**

A process for the preparation of 1,2-benzisothiazolin-3-ones of the formula

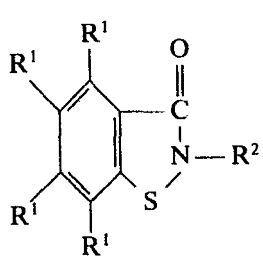

(I)

where the individual radicals $R^1$ may be identical or different and each denotes hydrogen, an aliphatic radical, an aromatic acyl group, a nitro group or halogen, it being possible for two adjacent $R^1$'s together with the two adjacent carbon atoms to be members of a fused aromatic ring, and $R^2$ is hydrogen or an aliphatic, cycloaliphatic, araliphatic or aromatic radical, wherein

(a) benz-1,2-dithiol-3-thiones of the formula

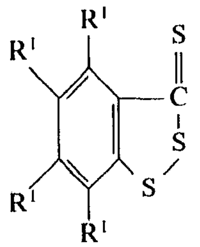

(II)

where $R^1$ has the above meanings, are reacted with chlorine and water in the presence of an organic solvent which is inert under the reactions conditions, and

(b) the resulting 2-chlorothioarylcarboxylic acid chlorides of the formula

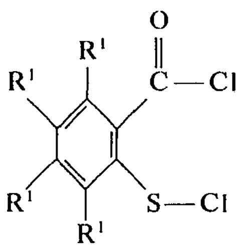

(III)

where $R^1$ has the above meanings, are reacted with ammonia or a primary amine of the formula

$$H_2N—R^2 \qquad \text{(IV)}$$

where $R^2$ has the above meanings, in the presence of an organic solvent which is inert under the reaction conditions.

**Revendication**

Procédé de préparation de 1,2-benzisothiazoline-3-ones de la formule

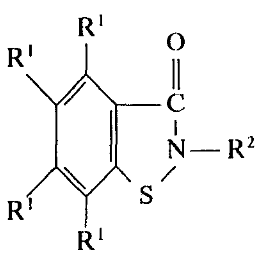

(I)

dans laquelle les divers substituants $R^1$, qui peuvent être identiques ou différents, désignent chacun un atome d'hydrogène, un groupe aliphatique, un groupe acyle aromatique, un groupe nitro ou un atome d'halogène, deux restes $R^1$ voisins pouvant en outre former avec les deux atomes de carbone adjacents des chaînons d'un noyau aromatique condensé, et $R^2$ désigne un atome d'hydrogène ou un groupe aliphatique, cycloaliphatique, araliphatique ou aromatique, caractérisé en ce que l'on fait réagir

a) des benzo-1,2-dithiol-3-thiones de la formule

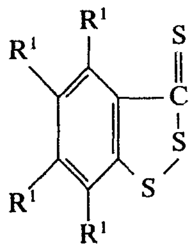

(II)

dans laquelle $R^1$ possède la signification définie, dans un solvant organique inerte dans les conditions réactionelles avec du chlore et de l'eau, puis

b) les chlorures d'acide 2-chloro-thio-aryl-carboxylique formés, de la formule

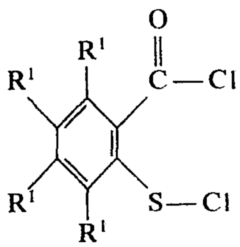

(III)

dans laquelle $R^1$ possède la signification définie, dans un solvant organique inerte dans les conditions opératoires avec de l'ammoniac ou une amine primaire de la formule

$$H_2N—R^2 \quad (IV)$$

dans laquelle $R^2$ possède la signification définie.